# EUROPEAN PATENT APPLICATION

(11) **EP 3 888 546 A1**
(43) Date of publication of application: **06.10.2021**
(21) Application number: 20167482.7
(22) Date of filing: 01.04.2020
(51) Int. Cl.: A61B 5/08, A61B 5/00

(54) **A SYSTEM AND METHOD FOR DETERMINING RESPIRATORY EFFORT**

(71) Applicant: Koninklijke Philips N.V., 5656 AG Eindhoven (NL)
(72) Inventor: DERKX, Rene Martinus Maria, 5656 AE Eindhoven (NL); VAN DE LAAR, Jakob, 5656 AE Eindhoven (NL); PRIORI, Rita, 5656 AE Eindhoven (NL)
(74) Representative: Philips Intellectual Property & Standards

(57) **Abstract**

The invention provides a system and method for determining a respiratory effort for a subject. The method comprises obtaining a relaxed signal representing the subject breathing in a relaxed manner and a forced signal representing the subject breathing in a forced manner. A plurality of forced peaks is derived from the forced signal and candidate peaks are selected from the plurality of forced peaks. The candidate peaks are selected based on features of the forced peaks. A user selects a user identified peak from the candidate peaks and thus, a respiratory effort is determined based on the relaxed signal and the user identified peak.

## Description

### FIELD OF THE INVENTION

The invention relates to systems and methods for determining the respiratory effort of a subject.

### BACKGROUND OF THE INVENTION

In subjects with chronic obstructive pulmonary disease (COPD) and other respiratory diseases, the assessment of the parasternal muscle activity (measured from surface electromyography (EMG), e.g. with electrodes positioned at the 2nd intercostal space) can be useful to estimate the intensity, timing and duration of subject respiratory effort, as an indicator of the balance between respiratory muscle load and respiratory muscle capacity. It is known from prior studies that the maximum EMG level that occurs during relaxed inhalation is related to the neural respiratory drive (NRD). In COPD subjects, during increasing lung hyperinflation as observed during acute exacerbation, there is a change in the balance between respiratory muscle load and capacity, which is reflected in the neural respiratory drive (lower capacity and higher load resulting in increased NRD). A way of assessing the NRD in a subject is to measure the respiratory effort of the subject.

In order to determine a normalized version of the respiratory effort, a sharp maximum inspiration through the nose is used. However, such maneuvers by themselves are known to have a large variance and may be potentially biased due to a lack of motivation or pain inhibition in affected subjects. Especially in clinical applications with pain-related inhibition (e.g. acute subjects) and elderly subjects, such full activation is difficult to achieve. Furthermore subjects might recruit other muscles during the maximum inspiratory maneuver, e.g. postural muscles, which act as a disturbance for the measurement and potentially elevate the maximum RMS values obtained during the maximum maneuver.

Therefore, there is a need to improve the way in which a respiratory effort is obtained.

### SUMMARY OF THE INVENTION

The invention is defined by the claims.

According to examples in accordance with an aspect of the invention, there is provided a system for determining a respiratory effort for a subject, the system comprising:
a processor configured to:
receive a relaxed signal from at least two electrodes, the relaxed signal representing a subject breathing in a relaxed manner;
receive a forced signal from the at least two electrodes, the forced signal representing a subject breathing in a forced manner;
derive a plurality of forced peaks from the forced signal;
select candidate peaks from the plurality of forced peaks, wherein a candidate peak is distinguished from a non-candidate peak based on features of the forced peaks;
obtain a user identified peak, wherein the user identified peak has been selected by a user; and
determine a respiratory effort based on the relaxed signal and the user identified peak.

The relaxed signal is obtained by asking the subject to breathe in a relaxed manner and measuring the signal from the electrodes. The forced signal is obtained by asking the subject to breathe in a forced, sharp manner (a sniff). Forced peaks can thus be derived from the forced signal (e.g. from an EMG waveform) at the corresponding time events. The forced peaks derived from the forced signal coincide with a sniff event. Some of the peaks may not be of appropriate quality due to various reasons (e.g. subject not breathing in as much as possible, subject stopping mid-sniff, subject being tired etc.). Therefore, candidate peaks are selected which correspond to "good" peaks which can be used to find the respiratory effort. The final selection of a user identified peak is made by a user. This provides a compromise between automated selection and user selection in that the user selects a peak from a limited set of peaks, in order to make the user involvement easier. Thereafter, the respiratory effort of the subject can be found based on the relaxed signal and the user identified peak.

The features of the forced peaks may be based on:
the maximum value of each of the forced peaks;
a sharpness indication, wherein the sharpness indication indicates the duration of a forced peak; and
a spectral flatness indication, wherein the spectral flatness indication indicates a comparison between the high frequency and low frequency components of the forced peak.

The maximum value of a forced peak corresponds to the peak amplitude of the forced peak. The sharpness indication is used to indicate the quality of a sniff. It corresponds to the duration of the peak and/or the effort exerted by the subject to perform the sniff. The sharpness, for example, may be calculated by calculating the derivative with respect to time of the forced peak, wherein the value of the derivative at the time of a sniff may be used to determine the sharpness indication.

The spectral flatness indication is also used to indicate the quality of a sniff. It corresponds to a comparison (e.g. ratio) of the contribution of high frequency components in the forced signal to the contribution of low frequency components at the time of a sniff. It may be calculated from using spectral analysis (e.g. applying Fourier transform) to the forced signal at the time of a sniff and, for example, determining the ratio of low frequency (e.g. < 200Hz) components in the spectral domain to the high frequency (e.g. > 200Hz) components in the spectral domain.

The system may further comprise a respiration sensor for monitoring movement or breathing flow during respiration, and wherein the processor is further configured to:
obtain a relaxed respiration signal based on at least one inspiratory manoeuver when the subject is breathing in a relaxed manner;
obtain a forced respiration signal based on at least one inspiratory manoeuver when the subject is breathing in a forced manner; and
select candidate peaks further based on the relaxed respiration signal and the forced respiration signal.

A second type of signal can also be used, a respiration signal. The respiration signal represents the physiological effects of breathing and can thus indicate the properties of an inspiratory manoeuver. The properties may include: whether the manoeuver corresponds to relaxed or forced breathing, the length of the manoeuver, the effort exerted by the subject during the manoeuver etc. For example, it can indicate the flow of air into the nose or the tilt of an accelerometer during breathing. The respiration signals can be used to determine whether a forced peak corresponds to a "good" sniff by, for example, comparing the corresponding respiration signal during the sniff to the respiration signal during relaxed breathing, or to respiration signals during previous sniffs.

The respiration sensor may be one or more of:
an accelerometer; and
a flow sensor.

The system may further comprise an output interface for providing feedback in real time for each of the forced peaks, wherein the feedback indicates one or more of:
whether a forced peak is selected as a candidate peak;
the number of candidate peaks currently selected;
based on a forced peak not being selected as a candidate peak, why the forced peak was not selected as a candidate peak; and
feedback on previous forced peaks.

The invention also provides a method for determining a respiratory effort for a subject, the method comprising:
obtaining a relaxed signal representing a subject breathing in a relaxed manner;
obtaining a forced signal representing a subject breathing in a forced manner;
obtaining a plurality of forced peaks from the forced signal;
selecting candidate peaks from the plurality of forced peaks based on features of the forced peaks;
obtaining a user identified peak, wherein the user identified peak has been selected by a user; and
determining a respiratory effort based on the relaxed signal and the user identified peak.

A feature of the forced peaks may comprise the values of each of the forced peaks and wherein selecting candidate peaks comprises comparing value of each of the forced peaks to one or more of:
values of the other forced peaks; and
a threshold forced peak value.

The method may further comprise obtaining a plurality of relaxed peaks from the relaxed signal, wherein selecting candidate peaks further comprises comparing each of the forced peaks to at least one of the relaxed peaks.

The method may further comprise:
obtaining a forced respiration signal based on at least one inspiratory manoeuver when the subject is breathing in a forced manner;
obtaining a plurality of forced inspiratory peaks based from the forced respiration signal;
wherein selecting candidate peaks is further based on comparing each of the forced inspiratory peaks to one or more of:
   the other forced inspiratory peaks; and
   a threshold forced inspiratory peak.

The method may also further comprise:
obtaining a relaxed respiration signal based on at least one inspiratory manoeuver when the subject is breathing in a relaxed manner; and
obtaining a plurality of relaxed inspiratory peaks based from the relaxed respiration signal,
wherein selecting candidate peaks is further based on comparing each of the forced inspiratory peaks to at least one of the relaxed inspiratory peaks.

Selecting candidate peaks may comprise:
determining a plurality of sharpness indications from the forced signal, wherein each sharpness indication indicates the duration of a forced peak and wherein a feature of a forced peaks comprises the corresponding sharpness indication; and
comparing each of the plurality of sharpness indications to one or more of:
   the other sharpness indications; and
   a threshold sharpness indication.

Selecting candidate peaks may comprise:
determining a spectral density of the forced signal for each of the forced peaks;
determining a high frequency spectral density from the spectral density based on frequencies above a threshold frequency;
determining a low frequency spectral density from the spectral density based on frequencies below the threshold frequency;
determining a spectral flatness indication for each of the forced peaks based on comparing the high frequency spectral density to the low frequency spectral density, wherein a feature of a forced peaks comprises the corresponding spectral flatness indication; and
comparing the spectral flatness indication for each of the forced peaks to one or more of:
   the other spectral flatness indications; and
   a threshold spectral flatness indication.

The forced signal may be obtained in real time and the candidate peaks may be selected in real time.

The method may also further comprise providing feedback in real time for each of the forced peaks, wherein the feedback indicates one or more of:
whether a forced peak is selected as a candidate peak;
the number of candidate peaks currently selected;
based on a forced peak not being selected as a candidate peak, why the forced peak was not selected as a candidate peak; and
feedback on previous forced peaks.

The invention also provides a computer program comprising code means for implementing the method defined above when said program is run on a processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows where on the body respiratory muscle activity may be measured;
Figure 2 three graphs representing signals from a subject breathing in a relaxed manner;
Figure 3 shows six graphs representing the EMG signals of a subject breathing;
Figure 4 shows a first example of a respiratory effort being determined;
Figure 5 shows an example of a method for determining a respiratory effort;
Figure 6 shows three graphs representing features of the peaks; and
Figure 7 shows a second example of a respiratory effort being determined.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for determining a respiratory effort for a subject. The method comprises obtaining a relaxed signal representing the subject breathing in a relaxed manner and a forced signal representing the subject breathing in a forced manner. A plurality of forced peaks is derived from the forced signal and candidate peaks are selected from the plurality of forced peaks. The candidate peaks are selected based on features of the forced peaks. A user selects a user identified peak from the candidate peaks and thus, a respiratory effort is determined based on the relaxed signal and the user identified peak.

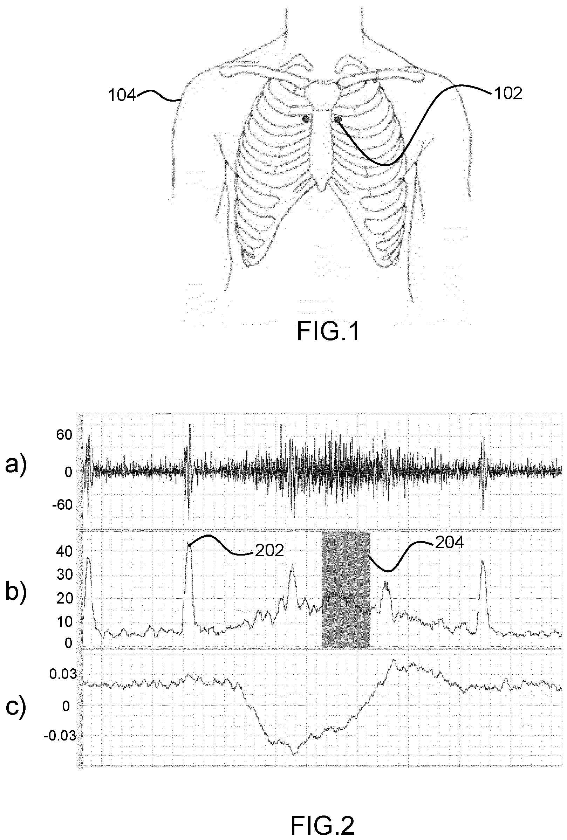

Figure 1 shows an example of the locations on the body of a subject 104 at which respiratory muscle activity can be measured. Two electromyogram (EMG) electrodes 102 located at the second intercostal space symmetrically near the sternum (parasternal) may be used for the measurement. The two electrodes 102 can be mounted inside (or attached on) a single EMG patch, which eases the placement of the two electrodes 102 to assess the same respiratory muscle groups for every sequential measurement (e.g. every day). It is known that the electrodes 102 mainly measure the inspiration breathing effort due to the activation of the parasternal internal intercostal muscles during inhalation by the subject 104.

Via the two EMG electrodes 102, the signal measured at the 2nd intercostal space parasternal muscle during inhalation can be used as an indicator of the day-to-day deterioration or improvement of the COPD subject 104 when multiple measurements are performed over a number of days, and as a predictor of hospital readmission after discharge as well.

However, signals from the respiratory muscle activity at the 2nd intercostal space also include electrocardiogram (ECG) signals from the heartbeat.

Figure 2 shows three graphs representing signals from a subject 104 during breathing.

The top graph a) shows the raw EMG and ECG signal (that includes the contribution from the electric activity of the heart - ECG contribution - that needs to be removed) during relaxed breathing of a COPD subject 104. The ECG contribution can be recognized by periodic strong peaks (may also be known as QRS complex).

The middle graph b) shows RMS values (in µV on the y-axis) from which it can be seen that the maximum RMS level in a particular single regular breath 204 is around 20 µV. This maximum RMS level corresponds to the maximum recruitment of the inspiratory parasternal muscle. The ECG RMS signal peaks 202 can also be seen in graph b).

The bottom graph c) shows the signal from a flow sensor that measures the pressure in the nose of a subject. A valley (negative pressure) in this signal indicates an inspiration.

When the maxima in the RMS signal is looked at during the relaxed breathing phase, there is the disadvantage that the levels of this RMS signal can be influenced by the level of subcutaneous skin tissue of the subject. It has also been seen from experiments that when subjects 104 are more obese, the RMS values are typically lower. Furthermore, it is known that the EMG amplitudes decrease with the distance between the electrode 102 and the muscle.

As a solution to this problem, the subject 104 can also perform a series of maximum effort maneuvers (in e.g. 1 minute) and measurements may be taken of the maximum RMS peak level during this maneuver. This allows the average of the RMS peak levels during the relaxed breathing to be divided (normalized) with the maximum of the RMS peak levels during maximum effort maneuvers. An important benefit of this normalization is to obtain a measurement during relaxed breathing that is presented as a percentage of respiratory muscle recruitment with respect to the maximum possible muscle recruitment. By having this result (e.g. a percentage) a threshold can more easily be defined in order to assess the subject 104 in terms of improvement or deterioration.

The highlighted area 204 in the middle graph b) shows the time when a subject 104 performed an inspiration. The inspiration EMG signal 204 are distinguished from the ECG signals 202 by a longer duration and a lower amplitude in the RMS signal. ECG signals 202 are periodic and have a sharp peak when compared to the inspiration EMG signals 204.

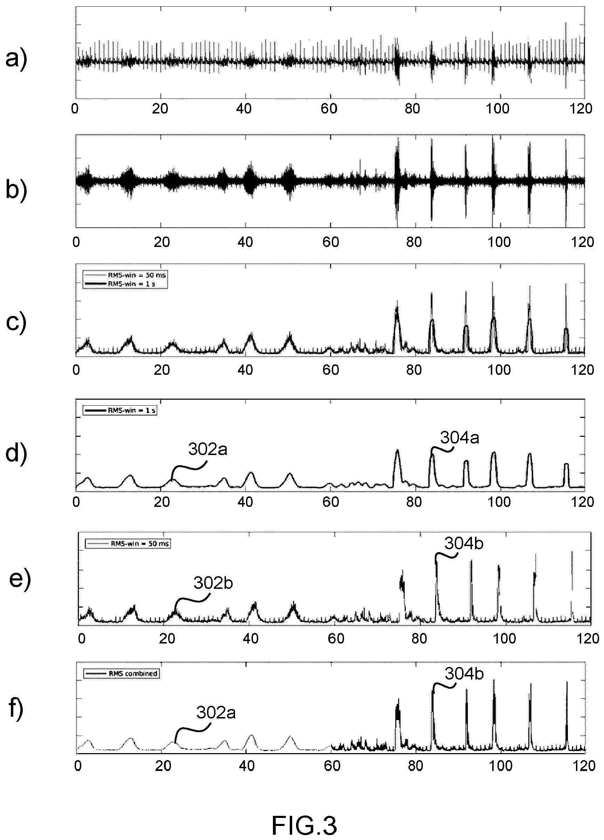

Figure 3 shows six graphs representing the EMG signals of a subject breathing.

The first graph a) shows the EMG and ECG signal. The ECG contribution is significant, since the signal is measured at the parasternal location. The x axis shows time in seconds.

The second graph b) shows a trace with the ECG contribution removed ("ECG-removed EMG"), where a 200 Hz high pass filter is used for the removal of the ECG contamination.

The third graph c) shows the RMS of the ECG-removed EMG, where two averaging windows are used for the computation of the RMS: a 50 ms averaging window and a 1 second averaging window.

The fourth graph d) shows the RMS of the ECG-removed EMG with an averaging window of 1 second.

The fifth graph e) shows the RMS of the ECG-removed EMG with an averaging window of 50 ms.

The sixth graph f) shows the RMS of the ECG-removed EMG with an averaging window of 1 second for the first 60 seconds and an averaging window of 50 ms for the last 60 seconds.

The 1 second averaging time is preferred in the relaxed breathing, because the 1 second RMS removes some ECG (residual) contamination and the 1 second RMS has a better defined peak level (better averaging of noise) for the relaxed signal. For example, the relaxed peak 302a has a better defined peak and lower noise level compared to 302b.

However, for the region of the sniffs (forced signal) in the last 60 seconds it can be seen that the RMS computation with 1 second averaging lowers the RMS levels during the sniffs maneuvers. This relates to the fact that the sniff is a sharp inspiratory maneuver that only has strongly activated parasternal respiratory muscles for a very short amount of time. Furthermore, it can be seen that for the 1 second RMS computation, sniffs with a longer duration (e.g. the first sniff maneuver at around 75 seconds) produce much higher RMS values compared to the sniffs with a short duration (e.g. the last sniff maneuver at around 115 seconds). This is not desired, since it would be preferable to have a reproducible maximum sniff level measurement that is independent of its duration. For example, the forced peak 304a has a much lower RMS peak level when compared to forced peak 304b.

Hence, there is not a unique choice possible for the RMS averaging windows that gives best output results for both the relaxed breathing and the sniff maneuvers.

It would be desirable to have a long averaging window for the RMS computation for relaxed breaths and short averaging window for the RMS computation for sniff maneuvers. Thus, it may be favorable to distinguish between RMS computations for the relaxed breaths and the sniff maneuvers, i.e. RMS with a first (long) averaging window (e.g. 1 second) for the relaxed breathing and RMS with second (short) averaging window (e.g. 50 ms) for the sniff maneuvers as is shown in graph f) of Figure 3.

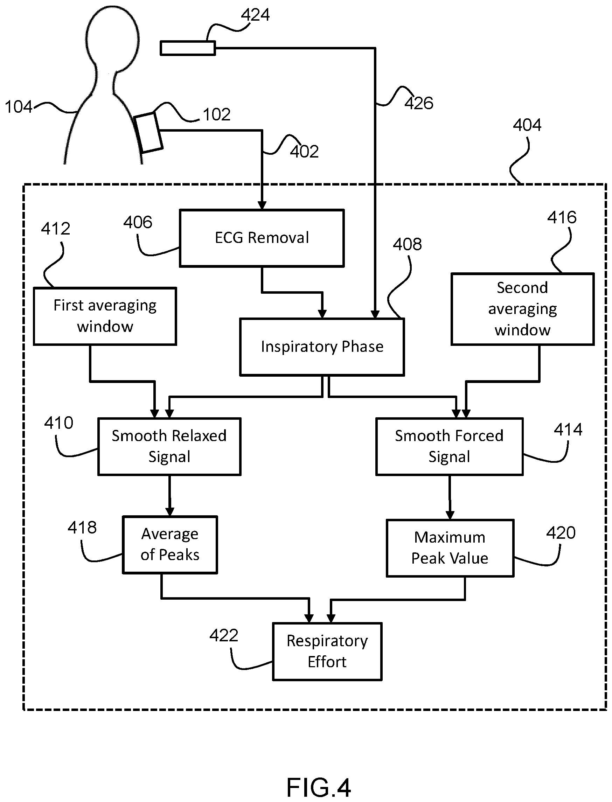

Figure 4 shows a first example of a system for determining a respiratory effort. A signal 402 is first obtained representing a subject breathing. The signal may be obtained from electrodes 102 on the subject 104 or from pre-recorded historic data for the subject 104. The signal 402 may cover time period during which the subject is breathing in a relaxed manner and/or the subject is breathing in a forced manner. The signal 402 is then processed by a processor 404.

Optionally, the signal 402 may first be filtered with an ECG removal block 406 if the signal 402 has not already been filtered. This will yield a filtered relaxed signal and a filtered forced signal. Since the EMG signal is measured on the parasternal area, there will be ECG contamination (ECG and EMG) in the signal. An ECG removal block 406 may thus be used on the EMG and ECG signal, where two types of ECG removal techniques may be applied:
(i) Spectral ECG removal, where a high pass filter is applied in the spectral domain with cutoff frequency of e.g. 200 Hz to effectively remove the ECG contribution, because it is known that the ECG contribution generally is minimal above 200 Hz; and
(ii) Temporal ECG removal, where a high pass filter is applied with cutoff frequency of e.g. 20 Hz to effectively remove the P- and T-waves from the ECG contribution and the remaining QRS complexes are removed via temporal masking.

The spectral ECG removal removes all spectral contributions of the ECG signal by having a high cut-off frequency. The temporal ECG removal retains the higher frequency spectral components in the frequency domain, but removes the high frequency QRS complexes based on the characteristic shape in the time domain, for example using a time-gated filtering technique, based on an ECG model.

Using either of these methods depends on the application, e.g. how much contribution to preserve in the frequency range of 20 to 200 Hz. For an easy implementation and robustness against arrhythmias, the spectral ECG removal may be used, since the detection of R-peaks and the construction of an ECG model are avoided.

The output of the ECG removal block 406 represents the signal that should be free of ECG contamination to such a level that is will not hamper the EMG measurement. In the inspiratory EMG block, the inspiratory phases 408 are selected from this ECG-removed signal.

The inspiratory phase 408 may comprise at least relaxed breathing and forced breathing, based on the subject breathing in a relaxed manner or performing sniffs.

Based on the inspiratory phase 408 being relaxed breathing, a smoothing function with a first (long) averaging window 412 is applied to the EMG signal in order to obtain a smoothed relaxed signal 410. Based on the inspiratory phase 408 being forced breathing (sniffs), a smoothing function with a second (short) averaging window 416 is applied to the EMG signal in order to obtain a smoothed forced signal 414.

The inspiratory phase 408 may be determined based on, for example, a nurse telling the subject 104 when to perform relaxed breathing and when to perform sniffs or by an automatic sniff detector.

The inspiratory phases 408 can be used as a guide to select the maximum peak 420 in the RMS of the ECG-removed signal. During relaxed breathing, a peak in every respiration cycle is selected and the average 418 over e.g. 1 minute may be computed. After the relaxed breathing, the subject 104 is asked to perform sniffs. The peak in each sniff maneuver is detected and the maximum 420 over all sniffs available (performed in e.g. 1 minute) may then be computed. The clinical EMG parameter that is computed is the respiratory effort 422 based on, for example, the average peak value 418 of the relaxed breathing which is normalized with (divided by) the maximum peak value 420 obtained from the sniff maneuver. In this way, a metric is obtained that represents a percentage of respiratory muscle recruitment with respect to the maximum possible muscle recruitment for the assessment of the subject in terms of improvement or deterioration.

The ECG and EMG signal may be buffered to collect samples in a block or a window of e.g. 10 seconds or 1 minute. Possibly the buffering also includes overlap with previous iterations to allow an output of 1 minute of data that is advanced with e.g. 10 seconds every iteration.

The ECG and EMG signal may alternatively be received from, for example, a memory module and further be processed (with different averaging windows). This may be for analyzing historical data of a subject. The signal received may also be pre-filtered (ECG signal removed).

Optionally the inspiratory phase 408 is determined with the help of a respiration unit 424 determining a respiration signal 426. For example, an accelerometer may be placed on the chest of the subject in order to measure the tilt of the chest. Alternatively, a flow sensor that measures the pressure in the nose of the subject may be used.

A respiration signal 426 may be obtained representing the subject breathing in a relaxed manner and the subject breathing in a forced manner, similar to how the EMG signal 402 is obtained. A plurality of peaks may be obtained from the forced respiration signal and from the relaxed respiration signal. The maximum value (or minimum value, depending on the direction of the peak) of the peaks may be representative of the quality of the sniff in the forced respiration signal. The maximum value (e.g. peak amplitude) of a forced respiration peak can be compared to the values of the other forced respiration peaks, to a threshold value or to the relaxed respiration peaks.

The respiration signal 426 may also be used to determine whether the EMG signal 402 is representative of the subject 104 breathing in a relaxed manner or a forced manner based on the duration, maximum value, minimum value and/or noise of the respiration signal 426. For example, if a flow sensor is used to determine the respiration signal 426, a relaxed breathing maneuver would cause a lower amount of flow than a sniff, such that the inspiratory phase 408 may be determined by the flow rate of the flow sensor.

An automatic sniff detection module may also be used. The automatic sniff detector may be able to detect, based on the respiration signal 426 and/or based on the EMG signal 402 whether the signal is a relaxed signal or a forced signal. This may be done with pre-calibration on a number of subjects, calibration on the subject 104 who generated the EMG signal 402 or with a pre-determined threshold voltage (or RMS voltage) in the EMG signal.

The smoothing function used, and corresponding averaging windows, may depend on the user (nurse) preference, the quality of the signals 402 obtained and/or on the processing equipment/software available. For example, a moving root mean square (RMS) may be used on both the relaxed signal and the forced signal, with the averaging window being the averaging window of the moving RMS, as has been shown in graphs c) d) e) and f) of Figure 3. Alternatively, a moving average may be computed for the signal or curve fitting may be used to approximate the signal.

In order to determine a most suitable averaging window, a set of smoothed signals with a variety of averaging windows may be computed and compared against each other. The suitability of the averaging window may depend on the judgment of the user (e.g. nurse) or on the average difference between data points of the real signal and the smoothed signal.

There may also be a user input interface for the user to input certain parameters. For example, the user may be able to input the type of smoothing function for the relaxed and forced signals, the durations of the first and second averaging windows and/or the ECG removal technique, as well as any further filtering required.

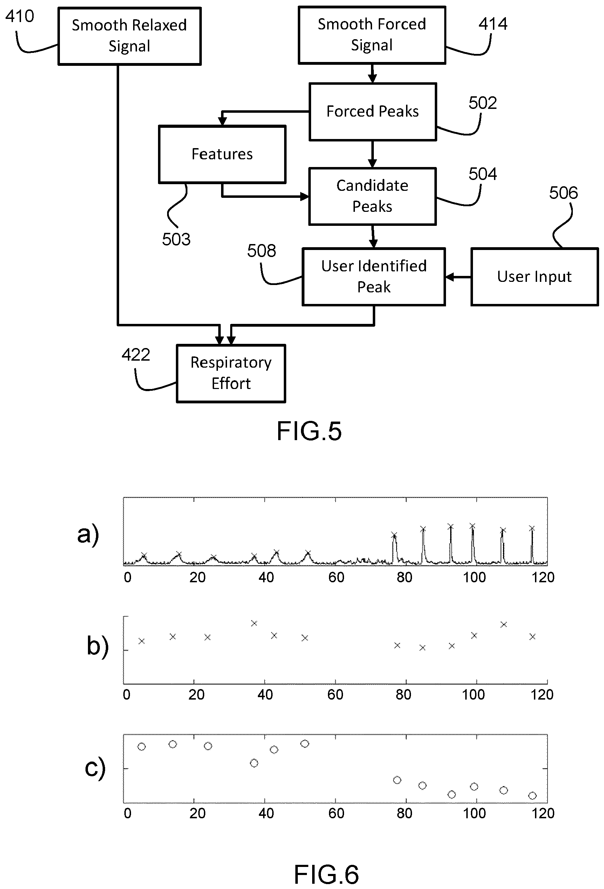

Figure 5 shows an example of a method for determining a respiratory effort 422. Since it is known know from several studies that a fully automated detection of sharp maximum inspiration maneuvers is very difficult to realize in practice, it is preferable to "guide" the nurse (performing the spot check respiratory effort measurement) in the procedure to obtain the best possible maximum inspiratory maneuver by the subject.

Forced peaks 502 can be first identified from the smoothed forced signal 414. A series of candidate peaks 504 can then be chosen based on the forced peaks 502 and features of the forced peaks 503. A user (e.g. nurse) can then select one of the candidate peaks through a user input 506 or a user identified peak 508, based on the judgment of the user. The user identified peak 508 and the smoothed relaxed signal 410 can then be used to calculate the respiratory effort 422.

For example, relaxed peaks can be identified from the smoothed relaxed signal 410 and the average value of the relaxed peaks can then be determined. The respiratory effort 422 can, for example, be obtained as the average relaxed peak value divided by the value of the user identified peak 508.

Also, the "series" of sniff maneuvers can be manually aborted by the user (e.g. nurse) when a valid sniff maneuver has been performed. Thus, the time spent by a subject 104 performing forced respiratory maneuvers (sniffs) can be minimized.

Figure 6 shows three graphs representing features 503 of the peaks.

The top graph a) shows the RMS signal of a high pass filtered (> 200 Hz) EMG signal. The x axis corresponds to time in seconds.

The middle graph b) shows the low to high (L/H) frequency component ratio, where the low frequency component is computed from 20 Hz up to 200 Hz of the EMG signal, whereas the high frequency component is computed from > 200 Hz of the EMG signal.

The bottom graph c) shows the duration of each inspiration.

Selection of candidate sniffs may be determined based on the measurement of some features 503 of the candidate sniff maneuvers (e.g. sharpness and flatness of the spectrum) and the logging of the history of the sniffs metrics. All this information can be provided to the nurse (in real time) to select the best possible sniff maneuver and abort the sniffs session when a good sniff maneuver has been obtained.

For example, the following features 503 may be used to determine the candidate sniffs:
(i) A sharpness indication which corresponds to the "sharpness" of the EMG signal (in time) during this maximum maneuver (e.g. the maximum maneuver should start at a low value of the RMS reading, quickly go to a high RMS value with a return to a low RMS reading with some reasonable time constant). For example, the sharpness indication could be determined from the first derivative of the RMS signals with respect to time.
(ii) A spectral flatness indication which corresponds to the "flatness" of the spectrum of the EMG signal during this maximum maneuver. The spectral flatness is based on the comparison of frequency components between two ranges. During inspiration bursts, the EMG spectrum is rather flat. However, when there is recruitment of contaminant (postural) muscles during a sniff maneuver, the spectrum will contain more low frequencies. Thus, the spectral flatness indication can de determined by measuring e.g. the ratio between the high frequency components (e.g. > 200 Hz) and the low frequency components (e.g. 20 up to 200 Hz). It can be seen that during poorly performed sniff maneuvers, this frequency ratio drops. The frequency components (high and low) may be derived from the measured power of each frequency range.

Larger values in the high to low frequency ratio, in graph b), represent either a higher proportion of high frequency components compared to low frequency components, which indicates a smaller recruitment of postural muscles during the sniff. During the first three sniffs (from 75 up to 95 seconds), it can be seen that the ratio is smaller compared to the last three sniffs (from 95 up to 120 seconds). Hence, the last three sniffs are considered to be performed better as they contain a lower proportion of the contaminating low frequencies from the postural muscles.

The duration of the EMG signal is shown in the lower graph, where it can be clearly seen that for the relaxed breaths (from 0 up to 60 seconds), the duration of the breath are much longer (less sharp) compared to the sniff maneuvers (from 60 up to 120 seconds). It can also be seen that the last 4 sniffs are more sharp (have shorter duration) compared to the first two sniffs.

Candidate peaks 504 can be selected based on a spectral flatness indication and a sharpness indication for each peak (e.g. in real time as the subject is performing the sniffs). Appropriate feedback can thus be communicated to the nurse in such a way that a next maximum effort maneuver can be done in a better way. For example, an automatic indication that a maneuver was not strong enough, was not done quickly enough (sharpness) or that the maneuver was not solely performed with the respiratory muscles alone (e.g. postural muscles recruited as well, giving not sufficient spectral flatness) can be communicated to the nurse, to further provide feedback to the subject.

The feedback may be communicated by an audiovisual output device, such as, for example, a display, a speaker, an interactive user interface or any combination thereof. The feedback provided may comprise whether the forced peak is selected as a candidate peak 504 based on the sharpness indication and/or the spectral flatness indication of the peak, the number of candidate peaks 504 which have been selected, why a forced peak 502 was not selected as a candidate peak 504 (e.g. took too long, used postural muscles, not strong enough etc.) and the features measured for each peak an how these features compare to the features of other forced peaks 502. The feedback on previous forced peaks 502 may also be displayed.

Based on the candidate peaks 504 and the feedback for each peak, the nurse can decide when to abort the sniff maneuvers session. Again, some guidance can be provided to the nurse to make this decision easier. For example, some information regarding last performed sniffs can be presented. In another example, the trend of the amplitudes or quality of the last performed sniff can be shown to easily observe that there is no room for improvement anymore. By early abortion of the sniffs session, unnecessary stress for the subject 104 can be avoided.

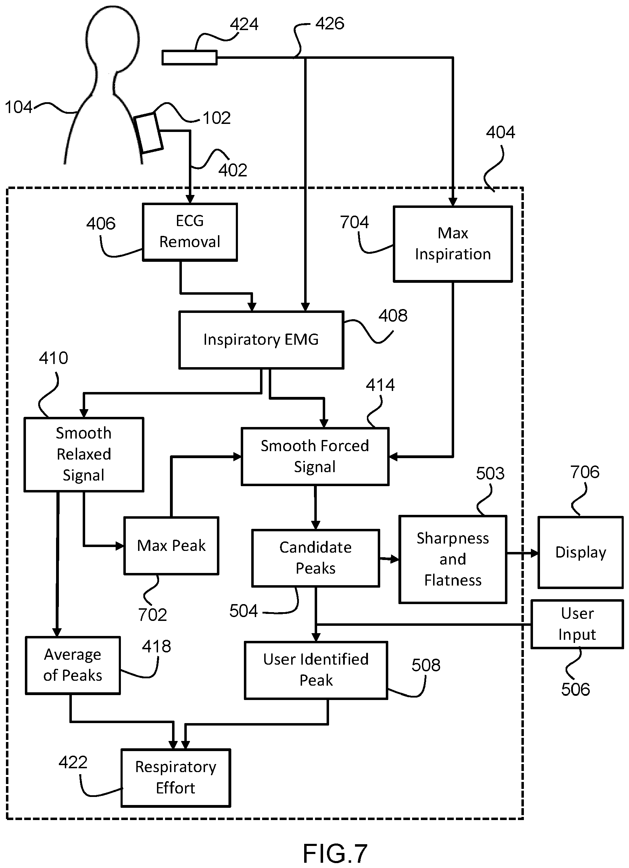

Figure 7 shows a second example of a system for determining a respiratory effort 422. For example, the electrodes 102 may record the EMG signal 402 for a few minutes (e.g. 3 minutes) focusing on obtaining the average of the relaxed phase RMS peak levels 418.

The smoothed relaxed signal 410 and the smoothed forced signal 414 are shown, but to prevent the figure being cluttered, the averaging windows (412 and 416) in Figure 4 are omitted.

Once the first few minutes have been passed, the average 418 and maximum 702 of the regular relaxed breaths are computed and the system goes automatically into the mode where the system tries to identify the RMS peaks during the maximum effort maneuvers (sniffs).

For the identification of the forced peaks from the smoothed forced signal 414, information obtained from the relaxed breathing phase (which is prior to the maximum effort breathing phase) can be used, for example:
(i) Comparing the RMS peak levels during the maximum maneuver with respect to an absolute threshold (minimum) RMS peak level or with respect to the RMS peak levels during the relaxed breathing, e.g. assuming that the RMS values of the maximum effort inspirations are at least 25% higher compared to the maximum of the RMS peak levels during the relaxed inspirations; and
(ii) Using the respiration signal 426 to detect a maximum maneuver with respect to an absolute (minimum) respiratory level (pressure or tilt) or with respect to respiratory peak levels during relaxed breathing 704, e.g. assuming that the maximum effort pressures are at least 25% higher compared to the maximum of the pressure peak levels 704 during relaxed inspirations. Alternatively, a maximum maneuver may be identified if the tilt of the accelerometer is for example at least 25% higher compared to the tilts during relaxed inspirations.

Candidate peak selection may also be based on the relaxed breathing phase. The features 503 (e.g. sharpness indication and spectral flatness indication) of the EMG signal 402 can be considered during the maximum inspiratory maneuver in order to provide information for the nurse regarding the quality of the sniff performed by the subject. The features 503 can be output to a display 706, such that the nurse can determine which of the candidate peaks 504 is most appropriate for the calculation of the respiratory effort 422.

Additionally, the smoothed relaxed signal 410, the relaxed signal, the smoothed forced signal 414, the forced signal, the respiration signal 426 and/or the candidate peaks 504 may be displayed on the display 706. The nurse can thus select the user identified peak 508 based on the candidate peaks 504 through a user input interface 506 based on the information on the display 706. Alternatively, the nurse may select the user identified peak 508 based on the performance of the maneuver performed by the subject 104 (e.g. sound, duration etc.) based on the judgment of the nurse.

The skilled person would be readily capable of developing a processor for carrying out any herein described method. Thus, each step of a flow chart may represent a different action performed by a processor, and may be performed by a respective module of the processing processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for determining a respiratory effort (422) for a subject (104), the system comprising a processor (404) configured to:
receive a relaxed signal representing a subject (104) breathing in a relaxed manner;
receive a forced signal representing a subject (104) breathing in a forced manner;
derive a plurality of forced peaks (502) from the forced signal;
select candidate peaks (504) from the plurality of forced peaks (502), wherein a candidate peak (504) is distinguished from a non-candidate peak based on features (503) of the forced peaks (502);
obtain a user identified peak (508), wherein the user identified peak (508) has been selected by a user from the candidate peaks; and
determine a respiratory effort (422) based on the relaxed signal and the user identified peak (508).

2. The system as claimed in claim 1, wherein the features (503) of the forced peaks (502) for the selection of the candidate peaks (504) comprise one or more of:
the maximum value of each of the forced peaks (502);
a sharpness indication, wherein the sharpness indication indicates the duration of a forced peak (502); and
a spectral flatness indication, wherein the spectral flatness indication indicates a comparison between the high frequency and low frequency components of the forced peak (502).

3. The system as claimed in claims 1 to 2, wherein the processor (404) is further configured to:
receive a relaxed respiration signal representing the movement or breathing flow of at least one inspiratory manoeuver when the subject (104) is breathing in a relaxed manner;
receive a forced respiration signal representing the movement or breathing flow of at least one inspiratory manoeuver when the subject (104) is breathing in a forced manner; and
select candidate peaks (504) further based on the relaxed respiration signal and the forced respiration signal, wherein the relaxed respiration signal and the forced respiration signal indicate the properties of the inspiratory manoeuvers.

4. The system as claim 3, further comprising a respiration unit (424) for obtaining the relaxed respiration signal and the forced respiration signal, and wherein the respiration unit (424) comprises one or more of:
an accelerometer; and
a flow sensor.

5. The system as claimed in any one of claims 1 to 4, further comprising an output interface (706) for providing feedback in real time for each of the forced peaks (502), wherein the feedback indicates one or more of:
whether a forced peak (502) is selected as a candidate peak (504);
the number of candidate peaks (504) currently selected;
based on a forced peak (502) not being selected as a candidate peak (504), why the forced peak (502) was not selected as a candidate peak (504);
the features (503) of one or more of the forced peaks (502): and
feedback on previous forced peaks (502).

6. The system as claimed in any one of claims 1 to 5, further comprising at least two electrodes (102) arranged to obtain the relaxed signal and/or the forced signal, when attached to the subject.

7. A method for determining a respiratory effort (422) for a subject (104), the method comprising:
receiving a relaxed signal representing a subject (104) breathing in a relaxed manner;
receiving a forced signal representing a subject (104) breathing in a forced manner;
deriving a plurality of forced peaks (502) from the forced signal;
selecting candidate peaks (504) from the plurality of forced peaks (502) based on features (503) of the forced peaks (502);
obtaining a user identified peak (508), wherein the user identified peak (508) has been selected by a user from the candidate peaks; and
determining a respiratory effort (422) based on the relaxed signal and the user identified peak (508).

8. The method as claimed in claim 7, wherein a feature (503) of the forced peaks (502) for the selection of the candidate peaks (504) comprises the maximum values of each of the forced peaks (502) and wherein selecting candidate peaks (504) comprises comparing the maximum value of each of the forced peaks (502) to one or more of:
the maximum value of the other forced peaks (502); and
a threshold forced peak value.

9. The method as claimed in any one of claims 7 or 8, further comprising obtaining a plurality of relaxed peaks from the relaxed signal, wherein selecting candidate peaks (504) further comprises comparing each of the forced peaks (502) to at least one of the relaxed peaks.

10. The method a claimed in any one of claims 7 to 9, further comprising:
receiving a forced respiration signal representing the movement or breathing flow of at least one inspiratory manoeuver when the subject (104) is breathing in a forced manner;
obtaining a plurality of forced inspiratory peaks from the forced respiration signal;
wherein selecting candidate peaks (504) is further based on comparing each of the forced inspiratory peaks to one or more of:
the other forced inspiratory peaks; and
a threshold forced inspiratory peak.

11. The method as claimed in claim 10, further comprising:
receiving a relaxed respiration signal representing the movement or breathing flow of at least one inspiratory manoeuver when the subject (104) is breathing in a relaxed manner; and
obtaining a plurality of relaxed inspiratory peaks based from the relaxed respiration signal,
wherein selecting candidate peaks (504) is further based on comparing each of the forced inspiratory peaks to at least one of the relaxed inspiratory peaks.

12. The method as claimed in any one of claims 7 to 11, wherein selecting candidate peaks (504) comprises:
determining a plurality of sharpness indications from the forced signal, wherein each sharpness indication indicates the duration of a forced peak (502) and wherein a feature (503) of a forced peaks (502) comprises the corresponding sharpness indication; and
comparing each of the plurality of sharpness indications to one or more of:
the other sharpness indications; and
a threshold sharpness indication.

13. The method as claimed in any one of claims 7 to 12, wherein selecting candidate peaks (504) comprises:
determining a spectral density of the forced signal for each of the forced peaks (502);
determining a high frequency spectral density from the spectral density based on frequencies above a threshold frequency;
determining a low frequency spectral density from the spectral density based on frequencies below the threshold frequency;
determining a spectral flatness indication for each of the forced peaks (502) based on comparing the high frequency spectral density to the low frequency spectral density, wherein a feature of a forced peaks (502) comprises the corresponding spectral flatness indication; and
comparing the spectral flatness indication for each of the forced peaks (502) to one or more of:
the other spectral flatness indications; and
a threshold spectral flatness indication.

14. The method as claimed in any one of claims 7 to 13, wherein the forced signal is obtained in real time and the candidate peaks (504) are selected in real time, and wherein the method further comprises providing feedback in real time for each of the forced peaks (502), wherein the feedback indicates one or more of:
whether a forced peak (502) is selected as a candidate peak (504);
the number of candidate peaks (504) currently selected;
based on a forced peak (502) not being selected as a candidate peak (504), why the forced peak (502) was not selected as a candidate peak (504); and
feedback on previous forced peaks (502).

15. A computer program comprising code means for implementing the method of any one of claims 7 to 14 when said program is run on a processing system.
